# EUROPEAN PATENT SPECIFICATION

(11) **EP 3 930 739 B1**
(45) Date of publication and mention of the grant of the patent: **16.07.2025**
(21) Application number: 20712209.4
(22) Date of filing: 25.02.2020
(51) Int. Cl.: A61K 36/185, A61P 19/00

(54) **MAQUI BERRY EXTRACTS FOR USE IN THE TREATMENT OR PREVENTION OF BONE DISORDERS**
MAQUI-BEEREN-EXTRAKTE ZUR VERWENDUNG IN DER BEHANDLUNG ODER PRÄVENTION VON KNOCHENERKRANKUNGEN
EXTRAITS DE BAIE DE MAQUI DESTINÉS À ÊTRE UTILISÉS DANS LE TRAITEMENT OU LA PRÉVENTION DE TROUBLES OSSEUX

(30) Priority: 27.02.2019 EP 19159847
(43) Date of publication of application: 05.01.2022
(73) Proprietor: Anklam Extrakt GmbH, 17389 Anklam (DE)
(72) Inventor: BONNLÄNDER, Bernd, 90571 Schwaig (DE); LANG, Stefanie, 90480 Nürnberg (DE); SUZUKI, Keiko, Yokohama Kanagawa 227-0066 (JP)
(74) Representative: Simandi, Claus
(86) International application number: PCT/EP2020/054951
(87) International publication number: WO 2020/173968

(56) References cited:
- EP-A1- 2 710 901
- WO-A2-02/17732
- WO-A2-2009/063333
- WO-A2-2011/048479
- JP-A- 2016 210 757
- WATSON ET AL: "Nutraceutical and antioxidant effects of a delphinidin-rich maqui berry extract DelphinolR: a review", MINERVA CARDIOANGIOL 2015 VOL. 63 -SUPPL. 1 TO NO. 2, 1 April 2015 (2015-04-01), pages 1 - 12, XP055608008, Retrieved from the Internet <URL:https://www.vivasaudavel.pt/fotos/gca/015maqui_delphinolschoenlau_1456322445.pdf> [retrieved on 20190723]
- VALERIA ROMANUCCI ET AL: "Bioactive Compounds of Aristotelia chilensis Stuntz and their Pharmacological Effects", CURRENT PHARMACEUTICAL BIOTECHNOLOGY, vol. 17, no. 6, 11 April 2016 (2016-04-11), NL, pages 513 - 523, XP055608012, ISSN: 1389-2010, DOI: 10.2174/1389201017666160114095246
- DATABASE WPI Week 201683, Derwent World Patents Index; AN 2016-770176, XP002793195
- SAWAKO MORIWAKI ET AL: "Delphinidin, One of the Major Anthocyanidins, Prevents Bone Loss through the Inhibition of Excessive Osteoclastogenesis in Osteoporosis Model Mice", PLOS ONE, vol. 9, no. 5, 13 May 2014 (2014-05-13), pages e97177, XP055607995, DOI: 10.1371/journal.pone.0097177

## Description

The present invention relates generally to maqui berry for use in the treatment or prevention of diseases or defects. In particular, the present invention relates to the use of maqui berry extract in the manufacture of a medicament or dietary supplement for use in treating or preventing of bone diseases or defects selected from the group of congenital bone defects, bone or spinal deformation, osteosarcoma, bone dysplasia, osteomalacia (rickets), osteogenesis imperfecta (brittle bone disorder), Paget's disease of the bone.

Bone is a living tissue comprising a number of constituents including calcium carbonate, calcium phosphate, collagen and water and is continuously being replenished by resorption and deposition of bone matrix. When a bone fractures the usual treatment is to reposition the fractured bone back into place, to stabilise the position of the bone, and then to wait for the bone healing process to occur. Bone healing is a complex process which is generally regarded as involving three phases: reactive phase, reparative phase and remodelling phase.

During the reactive phase a haematoma forms at the fracture site and inflammatory cells and fibroblasts infiltrate the bone under prostaglandin mediation. This results in the formation of granulation tissue, ingrowth of vascular tissue, and migration of mesenchymal cells. During the reparative phase, fibroblasts begin to lay down stroma that helps support vascular ingrowth. As vascular ingrowth progresses, a collagen matrix is laid down while osteoid is secreted and subsequently mineralised, which leads to the formation of a soft callus (cartilage) around the repair site. After a period of time from weeks to months the cartilage ossifies, forming a bridge of woven bone between the fracture fragments. During the remodelling phase the healing bone is restored to its original shape, structure, and mechanical strength. The remodelling phase occurs slowly over months to years; however, adequate bone strength is typically achieved in three to six months.

The maqui berry, also called Chilean wineberry (Aristotelia chilensis), is a native berry that only grows in the wild forests of Patagonia localized in Chile and Argentina. Up to now, almost no industrial cultivation does occur and most of the berries are still harvested from wild plants. The Patagonian region is close to the Andes Mountains and exhibits a quite harsh climate cold nights, warm days and intensive UV radiation. These conditions provide maqui berries with a unique phytochemical profile. The small deep purple to black berries have been consumed for centuries by the indigenous Mapuche Indians since the fruits are known for their remarkable health-promoting effects and valuable ingredients. Today next to goji and acai, the maqui berry is regarded as "super fruit" due to its superior antioxidant properties. In Chile, the berries are used for jam, juices, ice cream and liquors and are part of the daily life.

Growing evidence suggests that natural compounds in fruits and vegetables such as polyphenols have a wide range of biological and pharmacological effects. Among them, several flavonoids, a subgroup of polyphenols including hesperidin, quercetin and luteolin, have been shown to exert preventive efficacy for bone loss in ovariectomized animal models [Tsuji, M.; Yamamoto, H.; Sato, T.; Mizuha, Y.; Kawai, Y.; Taketani, Y.; Kato, S.; Terao, J.; Inakuma, T.; Takeda, E. Dietary quercetin inhibits bone loss without effect on the uterus in ovariectomized mice. J. Bone Miner. Metab. 2009, 27, 673-681, doi: 10.1007.]. Likewise, an anthocyanin-rich compound from blueberries was reported to possess protective property against bone loss in ovariectomized rat model [Devareddy, L.; Hooshmand, S.; Collins, J.K.; Lucas, E.A.; Chai, S.C.; Arjmandi, B.H. Blueberry prevents bone loss in ovariectomized rat model of postmenopausal osteoporosis. J. Nutr. Biochem. 2008, 19, 694-699.].

Watson et aL: "Nutraceutical and antioxidant effects of a delphinidin-rich maqui berry extract Delphinol®: a review", Minerva Cardioangiol 2015 Vol. 63 -Suppl. 1 to No. 2, 1 April 2015, pages 1-12 is a review describing the effects of Delphinol^{®} and it discloses that delphinidins suppress the differentiation and function of osteoclasts so that Delphinol^{®}, which is rich in delphinidins, has a property of preventing bone demineralization.

Current anti-osteoporotic drugs, such as several bisphosphonates, anti-RANKL antibodies and selective estrogen receptor modulators, have been widely used and their bone protective effects are well-established. These drugs however, are anti-resorptive acting by inhibiting osteoclast activity, which may lead to suppressed bone remodelling. Thus, microdamage developing in bone due to a lack of the repair process is thought to be the reason why fractures occur in a substantial proportion of osteoporotic patients even though they may have reached normal bone mineral density (BMD) after treatment. In order to maintain bone health and ensure mobility, even after longitudinal bone growth stops, adequate amounts of new bone need to be formed following bone resorption.

Thus, it is an object of the invention to provide drugs that are useful for treatment or prevention of diseases or defects selected from the group of congenital bone defects, bone or spinal deformation, osteosarcoma, bone dysplasia, osteomalacia (rickets), osteogenesis imperfecta (brittle bone disorder), Paget's disease of the bone.

The present inventors have surprisingly found that maqui berry extract disclosed herein promote bone repair by inducing new bone formation.

Hence, disclosed herein is a method for use in the treatment of bone in a subject, the method comprising administering to the subject in need thereof an effective amount of an maqui berry extract as defined herein.

The subject may be suffering from a defect or disease or may be susceptible or predisposed to such defect or disease. TThe disease may be a bone disease, particularly selected from the group of congenital bone defects, bone or spinal deformation, osteosarcoma, bone dysplasia, osteomalacia (rickets), osteogenesis imperfecta (brittle bone disorder), Paget's disease of the bone.

All mentioned diseases and defects are well described in Pschyrembel (e.g. https://www.pschyrembel.de/).

The maqui berry extract may enhance the rate or extent of healing of the disease.

The defect or disease may be acute or chronic. Those skilled in the art will readily appreciate the scope of defects and diseases to which the embodiments disclosed herein relate, being those in which the formation of new bone, the repair of damaged or otherwise defective bone, or the regeneration of bone is desirable or advantageous. In a preferred embodiment, said defects and diseases are selected from the group of congenital bone defects, bone or spinal deformation, osteosarcoma, bone dysplasia, osteomalacia (rickets), osteogenesis imperfecta (brittle bone disorder), Paget's disease of the bone, characterised by or associated with abnormal bone metabolism, formation or resorption.

Therefore, an approach, especially with regard to antioxidative protection of maqui berry extract, is important for primary prevention of bone loss, which may in turn lower the risk of fractures in later life and improve the healing procedures of the disease. Moreover, it is advantageously that maqui berry extract shows anti-inflammatory effects.

The maqui berry extract can be obtained by means of water/alcohol and other solvents based on each obtained fraction from a raw material, preferably fruits and berries. Such methods are well known in the state of the art.

The aqueous/alcoholic extraction agent is used in the ratio of 40 : 60 (v/v) to 60 : 40 (v/v), in particular 41 : 59 (v/v), or 50 : 50 (v/v).

In particular ethanol, in particular 80% ethanol (v/v) or more, like 96 % ethanol (v/v), is used as alcohol in all embodiments of the invention.

According to the invention the maqui berry extract is enriched with anthocyanins (containing sugar), in particular delphinidin glycoside (so called delphinidins) as an active substance. In particular, the maqui berry extract is enriched with delphinidin-3-sambubioside-5-glucoside, delphinidin-3,5-diglucoside, delphinidin-3-sambubioside or delphinidin-3-glucoside. Moreover, delphinidin-3-O-galactoside, delphinidin 3-O-glucoside, cyanidin-3-O-galactoside, delphinidin-3-O-arabinoside, cyanidin-3-O-glucoside, petunidin-3-O-galactoside, petunidin-3-O-glucoside, cyanidin-3-O-arabinoside, peonidin-3-O-galactoside, petunidin-3-O-arabinoside, malvidin-3-O-galactoside, peonidin-3-O-glucoside, malvidin-3-O-glucoside, peonidin-3-O-arabinose, malvidin-3-O-arabinose are preferred compounds of the maqui berry extract.

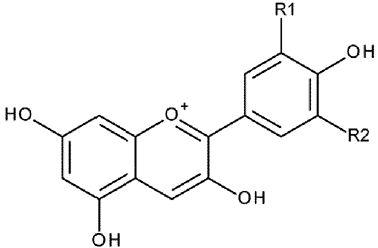

| R1 | R2 | anthocyanidin |
|---|---|---|
| OH | OH | delphinidin |
| OH | H | cyanidin |
| H | H | pelargonidin |
| OCH₃ | H | peonidin |
| OCH₃ | OH | petunidin |
| OCH₃ | OCH₃ | malvidin |

Fig. A - some anthocyanidin (sugar free)

According to the invention the maqui berry extract will be obtained by the following steps:
a) providing a crude extract of a plant material,
b) filtering said crude extract;
c) contacting said crude extract with an adsorbent, preferably a resin, like polymer resin, in particular a polystyrene resin, wherein said resin adsorbs said anthocyanins;
d) washing said adsorbent, in particular a resin; and
e) eluting said anthocyanins from said adsorbent to obtain a composition enriched for anthocyanins.

Hence, the present invention refers to a maqui berry extract standardized comprising a minimum of 25% delphinidins (w/w) and a minimum of 35% total anthocyanins (w/w).

In a preferred embodiment water is used for step d.). In a further preferred embodiment ethanol is used for step e.).

In a preferred embodiment the plant material for step a.) is selected from fruits and / or berries.

Such an obtained extract has a special and unique composition of ingredients due to the above-mentioned process-steps.

As used herein the terms "treating" and "treatment" or "preventing" and "prevention" refer to any and all uses which remedy a condition or symptoms, prevent the establishment of a condition or disease, or otherwise prevent, hinder, retard, or reverse the progression of a condition or disease or other undesirable symptoms in any way whatsoever. Thus, the terms "treating" and "treatment" or "preventing" and "prevention" are to be considered in their broadest context. For example, treatment does not necessarily imply that a subject is treated until total recovery.

As used herein the term "subject" includes humans and animals. Typically, the subject is a human, or a patient.

Preferably, the maqui berry extract according to the invention is formulated as a pharmaceutical composition comprising in addition one or more pharmaceutically acceptable carriers or diluents.

The medicinal drugs that are manufactured with maqui berry extract in accordance with the invention can be administered orally, parenterally, topically, intramuscularly, peri-articularly, intra-articularly, intravenously, intraperotoneally, subcutaneously, or rectally. The invention pertains to processes for the manufacture of medicinal drugs that are characterized by the feature that maqui berry extract according to the invention is/are brought into a suitable form of agent for administration together with a pharmaceutically suitable and physiologically tolerated vehicle and, optionally, further suitable active substances, additives, or ancillary substances. Suitable solid or liquid galenic forms of preparation or formulations are, for example, granulated materials, powders, sugar-coated pills, tablets, (micro)capsules, suppositories, syrups, juices, suspensions, emulsions, drops, or injectable solutions as well as preparations with a protracted release of the active substance, whereby use is made in their preparation of conventional ancillary substances, such as vehicle substances, agents that lead to the disintegration of the preparation, binders, coating agents, swelling agents, slippage promoting agents or lubricants, taste improving agents, sweeteners, and solubilizers. Mention may be made of the following as ancillary substances: magnesium carbonate, titanium dioxide, lactose, mannitol and other sugars, talcum, milk protein, gelatine, starch, cellulose and its derivatives, animal and vegetable oils such as cod-liver oil, sun flower oil, groundnut [oil] or sesame oil, poly(ethylene glycols), and solvents such as, for example, sterile water and monohydric or polyhydric alcohols, e.g. glycerine.

When the maqui berry extract is used in the preparation of pharmaceutical drugs, it may be administered in any form conveniently employed for oral or parenteral administration, such as injections (emulsifiable, suspendable, non-aqueous, etc.), or solid injections emulsified or suspended prior to use, transfusion solutions, powders, granules, tablets, capsules, enteric coated tablets, troches, liquid for internal use, suspensions, emulsions, syrups, liquids for external use, fomentations, nasal drops, inhalants, ointments, lotions, suppositories, enteral nutrients, etc. It may be used either alone or in combinations depending on the disease conditions. These may be prepared according to the conventional methods by adding to the main drug pharmacologically and pharmaceutically acceptable adjuvants for manufacture.

The medicinal drugs are preferably manufactured and administered in dosage units, whereby each unit contains, as the active component, a defined dose of the maqui berry extract according to the invention. In the case of solid dosage units, such as tablets, capsules, sugar-coated pills or suppositories, this dose can amount to 1 to 1000 mg and preferably 50 to 300 mg, and in the case of injection solutions in ampoule form, this dose can amount to 0.3 to 300 mg and preferably 10 to 100 mg.

Daily doses of 20 to 1000 mg of active substance, and preferably 100 to 500 mg of active substance, are indicated for the treatment of an adult patient weighing 50 to 100 kg, e.g. 70 kg. However, higher or lower daily doses can also be applied under certain circumstances. The administration of the daily dose can take place via an administration on one single occasion in the form of an individual dosage unit or several smaller dosage units, or via the multiple administration of subdivided doses at defined intervals.

In the following, the present invention is described in more detail by way of examples. However, these examples are not intended to limit the scope of protection of the present invention in any way.

The examples also refer to several figures, the legends of which are given below:

### Examples:

Delphinol^{®}, also under the brand name maquisupreme^{®}, is a highly water-soluble powdery extract of maqui berries produced under GMP conditions by Anklam Extrakt GmbH, Germany. Delphinol^{®} is standardized to contain a minimum of 25% delphinidins and a minimum of 35% total anthocyanins (supra).

### Example 1:

### Effects of Delphinol^{®} on osteoblast differentiation and functionality

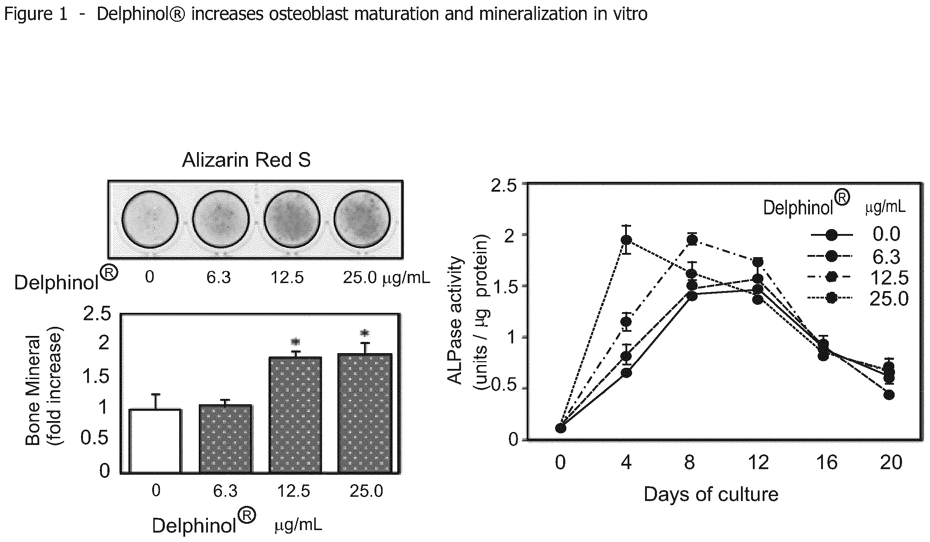

Using Delphinol^{®} on osteoblastic MC3T3-E1 cells in vitro it could be shown the extract stimulates multilayer proliferation und thus resulting in a siginifcant increase in mineral deposition in a dose-dependent manner detected with Alizarin red S staining of culture (Figure 1, left panels). In the course of bone nodule formation, Delphinol^{®} significantly stimulated actvity of osteoblastic marker enzyme ALP (Figure 1, right panel).

### Example 2:

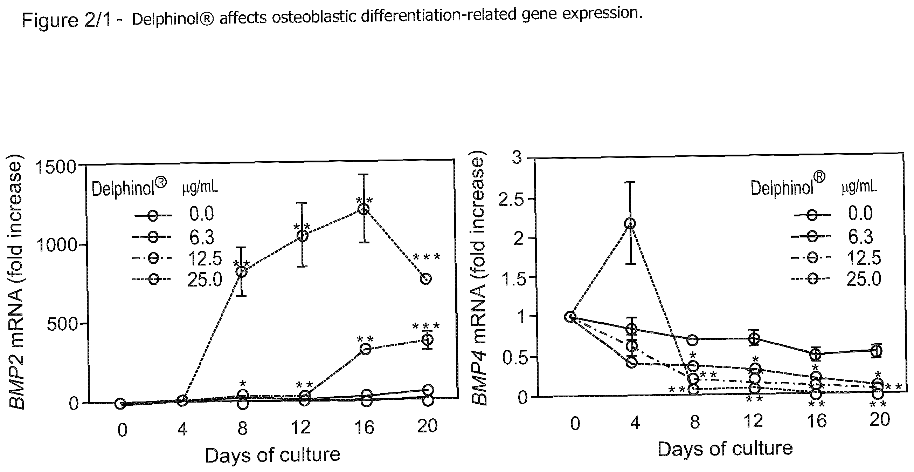

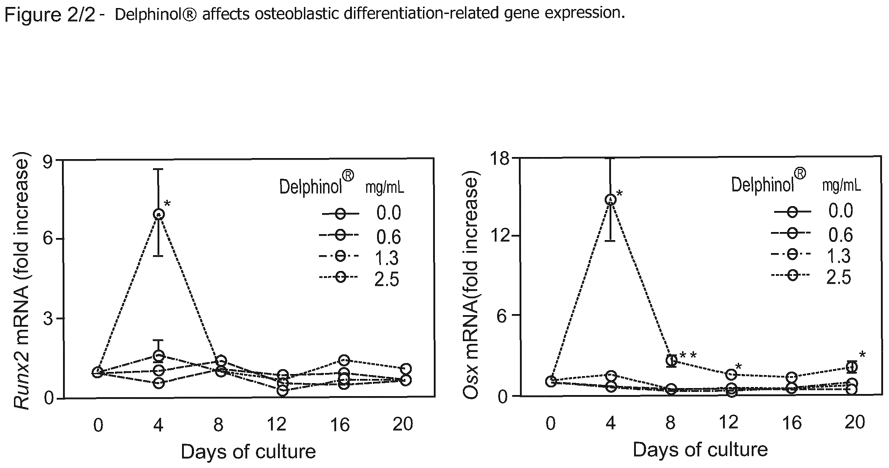

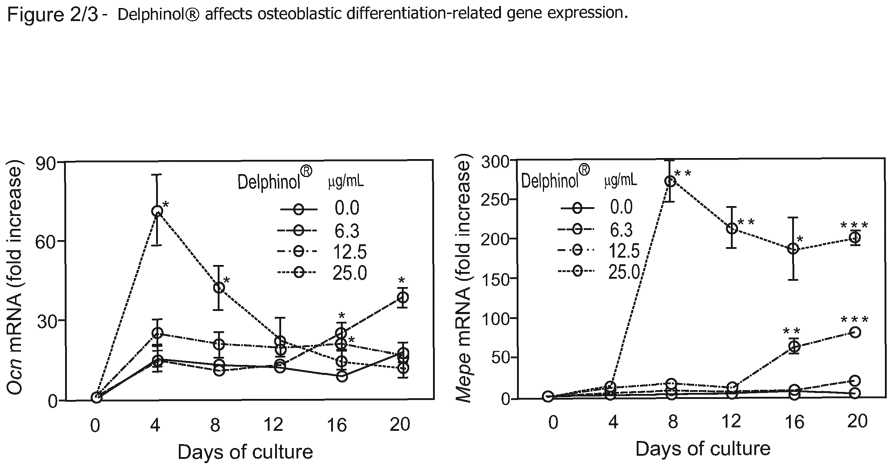

In addition Delphinol^{®} upregulated osetoclastic differentiation-specific genes, namely bone morphogenetic protein (Bmp) 2, Bmp4, Runx2 and Osx, two bone-specific transcription factors known to regulate bone homeostasis, matrix extracellular phosphoglycoprotein (Mepe), an extracellular matrix in bone and known to play key roles in tissue mineralization, and osteocalcin (Ocn), also known as bone γ-carboxy glutamic acid protein (bone gla protein) and a marker of bone formation (Figure 2).

### Example 3:

Protective and anabolic effects of oral administration of Delphinol^{®} against bone loss in two in vivo mouse models.

To verify the in vitro findings on bone building and mineralizing osteoblasts in an in vivo approach two osteopenic mouse models, namely ovariectomy-induced (OVX) as well as sRANKL-induced bone loss model, were conducted and femoral bone architecture analyzed with micro-CT scans in accordance to the methods of the ASBMR Histomorphometry Nomenclature Committee (Dempster et al., 2013 https://www.ncbi.nlm.nih.gov/pubmed/23197339).

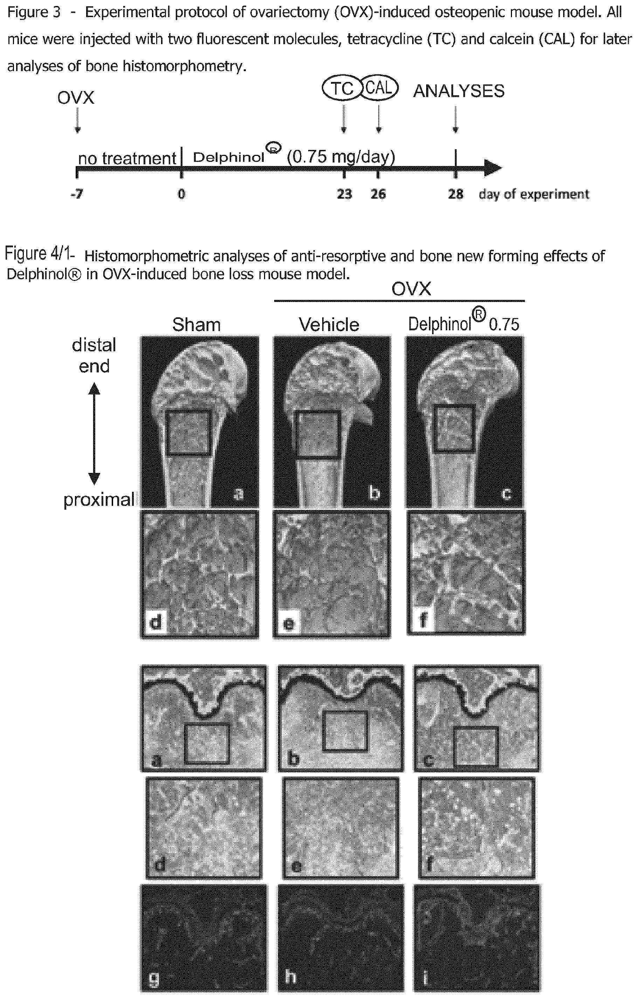

For the OVX-induced model, female mice were ovariectomized or sham-operated. Starting seven days after surgery, ovariectomized mice received either Delphinol^{®} (0.75mg/day) or inactive vehicle orally for in total 28 days. On days 23 and 26 two fluorescent molecules (tetracycline and calcein) were injected to enable analyses of bone microarchitecture on femurs (Figure 3).

### Example 4:

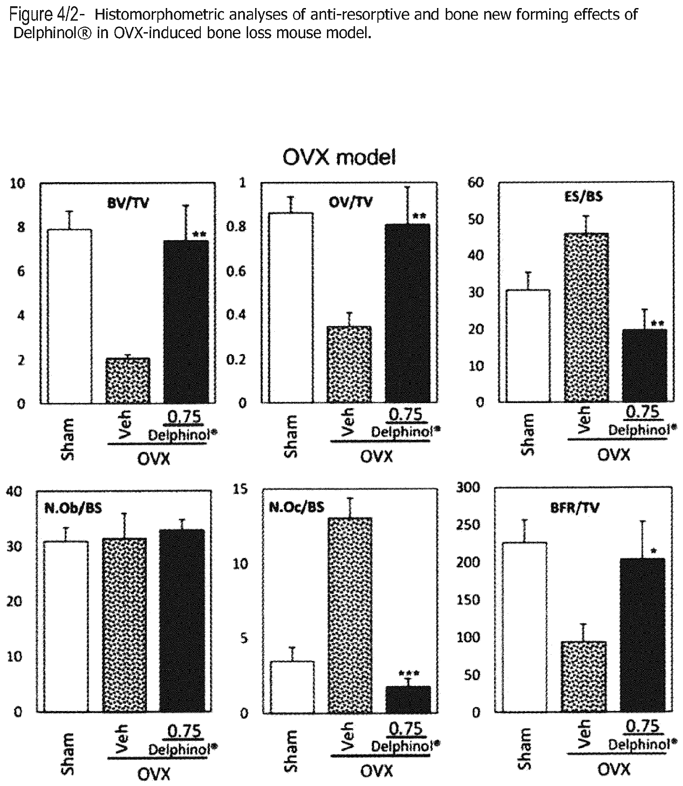

The three-dimensional sagittal midsection of distal femurs constructed from micro-CT scans demonstrated that trabecular bone, observed in Delphinol^{®} treated group was thicker than that of the vehicle or sham group mice, indicating that Delphinol^{®} could not only inhibit bone resorption, as seen in significantly decreased numbers of osteoclasts (N.Oc/BS), higher bone volume (BV/TV) and less eroded surface (ES/BS) but also accelerates bone formation (BFR/TV) and osteoid volume (OV/TV) consistent with the in vitro findings (Figure 4).

### Example 5:

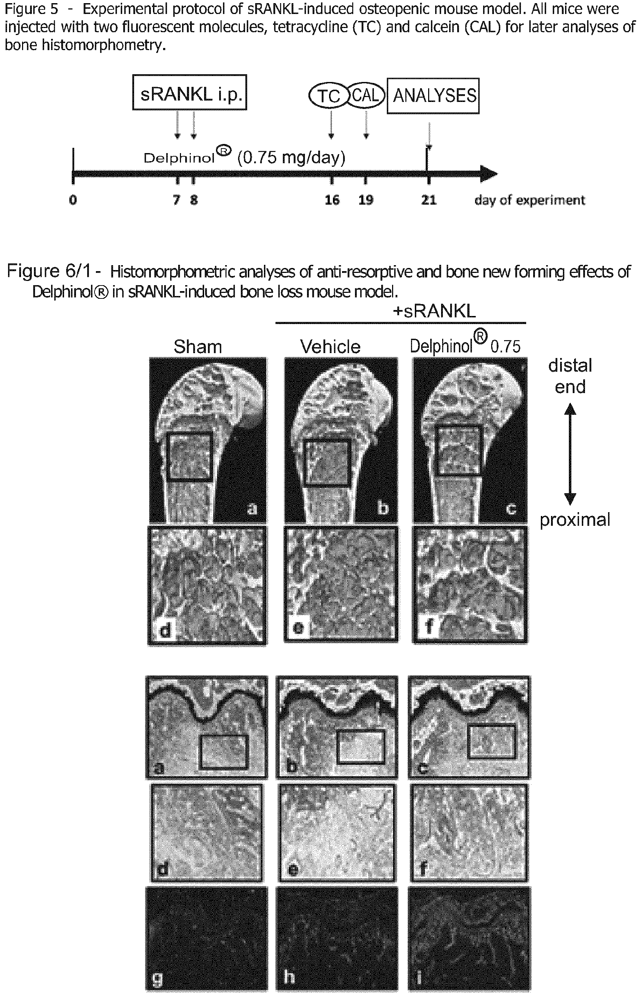

For the second osteopenia mouse model, female mice grouped in either untreated control group, vehicle group or Delphinol^{®} group. Latter group was administered with 0.75mg Delphinol^{®} each for 21 days. On days 7 and 8 sRANKL was injected intraperitoneal to the vehicle and Delphinol^{®} group. As in the OVX-model two fluorescent molecules, tetracycline (TC) and calcein (CAL), were injected at days 16 and 19 for later examination of bone microarchitecture (Figure 5).

### Example 6:

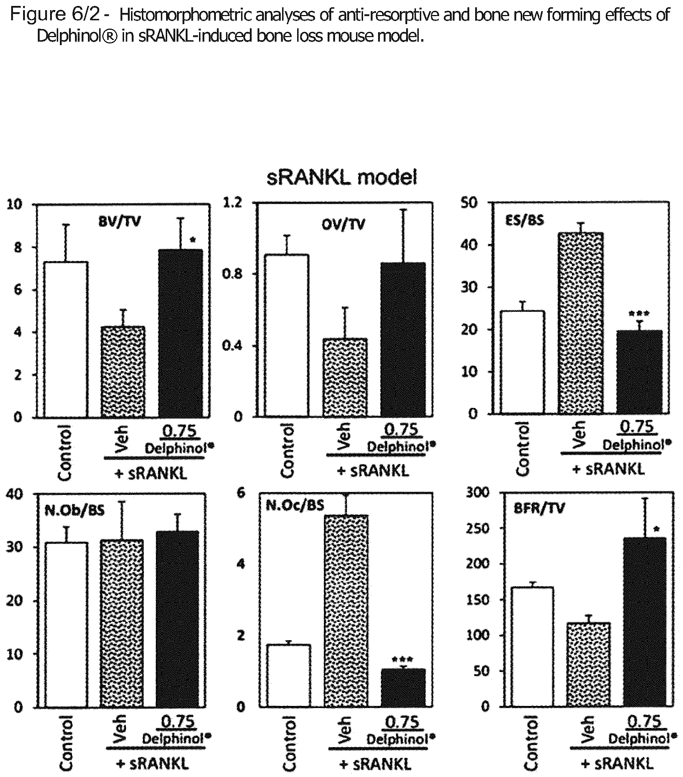

The three-dimensional sagittal midsection of distal femurs constructed from micro-CT scans demonstrated that trabecular bone, observed in Delphinol^{®} treated group was thicker than that of the vehicle or untreated control group mice, indicating that Delphinol^{®} could not only suppress bone decline, as seen in significantly decreased numbers of osteoclasts (N.Oc/BS), enhanced bone volume (BV/TV) and decreased eroded surface (ES/BS) but also stimulates bone formation (BFR/TV) and osteoid volume (OV/TV) consistent with the in vitro findings (Figure 6).

### Example 7:

### Effects of Delphinol^{®} on inflammatory NF-κB pathway

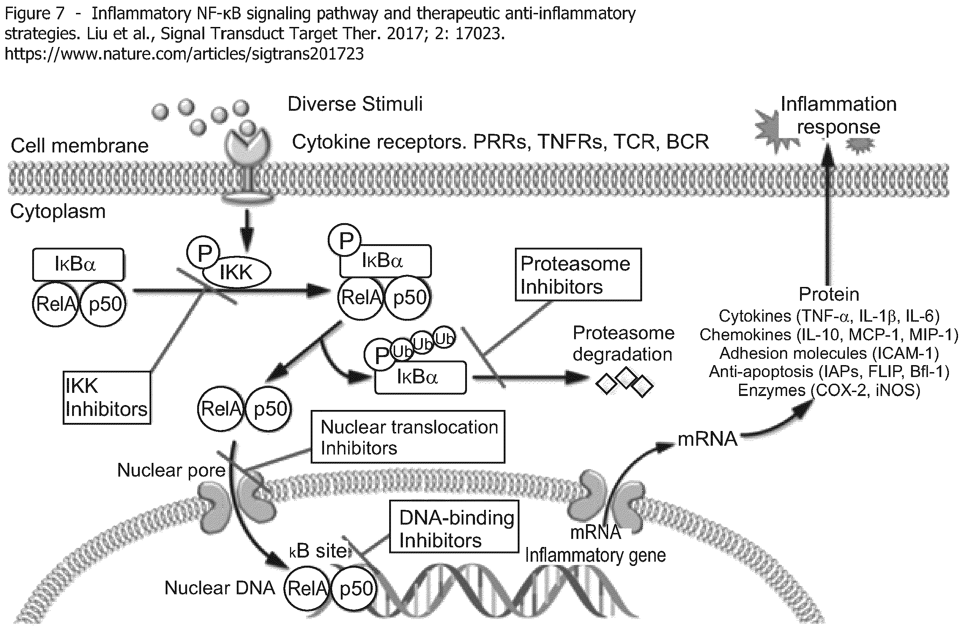

NF-κB is a well-known and intensively examined central inflammatory mediator that interacts with a broad variety of immune receptors and can also be stimulated by oxidative stress. Once activated, NF-κB in turn translocates from the cytoplasm into the nucleus and induces the expression of numerous pro-inflammatory genes, e.g. coding for cytokines and chemokines, and can in this way contribute to pathogenic processes in inflammatory diseases (Figure 7).

### Example 8:

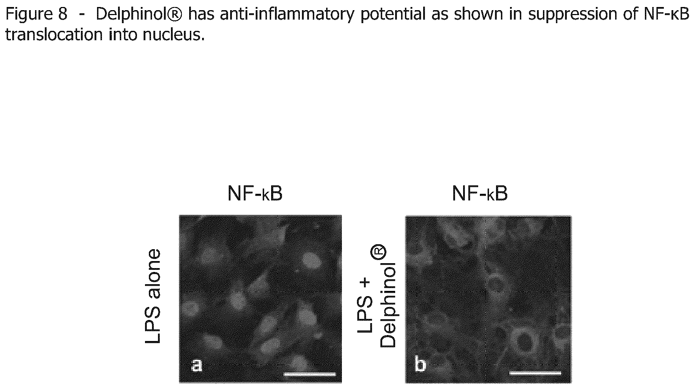

Examination of the influence of Delphinol^{®} on NF-κB active subunit p65 nuclear translocation revealed that Delphinol^{®} inhibited LPS-induced translocation of the p65 subunit into cell nuclei almost completely, which is clearly shown by the cytoplasmic staining (Figure 8) of osteoblastic MC3T3-E1 cells. LPS (a bacterial compound) was used to immune-stimulate the cells and known to induce strong oxidative stress as well as activating NF-κB inflammatory signaling cascade.

## Claims

1. Medicament or dietary supplement for use in the treatment or prevention of a bone disease or defect selected from the group of congenital bone defects, bone or spinal deformation, osteosarcoma, bone dysplasia, osteomalacia (rickets), osteogenesis imperfecta (brittle bone disorder), Paget's disease, comprising maqui berry extract, wherein the maqui berry extract comprises a minimum of 25% (w/w) delphinidins and a minimum of 35% (w/w) anthocyanins, and wherein the extract is obtained by the following steps:
a) providing a crude extract of a plant material,
b) filtering said crude extract;
c) contacting said crude extract with an adsorbent, wherein said adsorbent, in particular resin adsorbs said anthocyanins;
d) washing said adsorbent; and
e) eluting said anthocyanins from said adsorbent to obtain a composition enriched for anthocyanins.

2. Medicament or dietary supplement for use according to one of the preceding claims, wherein the maqui berry extract is obtained by an aqueous/alcoholic extraction agent, in particular 40 : 60 (v/v) to 60 : 40 (v/v), in particular 41 : 59 (v/v), or 50 : 50 (v/v).

3. Medicament or dietary supplement for use according to claim 2, wherein the alcoholic extraction agent is ethanol, in particular 96 % ethanol.

4. Medicament or dietary supplement for use according to one of the preceding claims, wherein water is used for step d.) and ethanol is used for step e.).

5. Medicament or dietary supplement for use according to one of the preceding claims, wherein the plant material of step a.) is selected from fruits.

6. Medicament or dietary supplement for use according to one of the preceding claims, wherein said adsorbent of steps c.) to d.) is a resin, in particular a polymer resin or a polystyrene resin.

7. Medicament or dietary supplement for use according to one of the preceding claims comprising a pharmaceutical preparations, where applicable including suitable carrier substances, in particular in the form of dragées, tablets, film-coated tablets, powders, capsules or liquid dilutions, in particular drops, juices or syrups, ointments, emulsions, granulates, powders, nasal sprays, liquid or solid preparations for inhalation, compresses, wound and gum dressings, tamponades, tonsil paint solutions, gargling solutions, rinsing solutions, injections, transfusion solutions, granules, enteric coated tablets, troches, suspensions, fomentations, nasal drops, inhalants, lotions, suppositories, enteral nutrients.

## Patentansprüche

1. Arzneimittel oder Nahrungsergänzungsmittel zur Verwendung bei der Behandlung oder Vorbeugung einer Knochenkrankheit oder eines Knochendefekts, ausgewählt aus der Gruppe der angeborenen Knochendefekte, Knochen- oder Wirbelsäulendeformation, Osteosarkom, Knochendysplasie, Osteomalazie (Rachitis), Osteogenesis imperfecta (Glasknochenkrankheit), Paget-Krankheit, umfassend Maqui-Beeren-Extrakt, wobei der Maqui-Beeren-Extrakt mindestens 25 % (Gew./Gew.) Delphinidine und mindestens 35 % (Gew./Gew.) Anthocyane umfasst, und wobei der Extrakt durch die folgenden Schritte erhalten wird:
a) Bereitstellen eines Rohextrakts aus einem Pflanzenmaterial,
b) Filtrieren des Rohextrakts;
c) Inkontaktbringen des Rohextrakts mit einem Adsorptionsmittel, wobei das Adsorptionsmittel, insbesondere Harz, die Anthocyanine adsorbiert;
d) Waschen des Adsorptionsmittels; und
e) Eluieren der Anthocyanine aus dem Adsorptionsmittel, um eine mit Anthocyanen angereicherte Zusammensetzung zu erhalten.

2. Arzneimittel oder Nahrungsergänzungsmittel zur Verwendung nach einem der vorhergehenden Ansprüche, **dadurch gekennzeichnet, dass** der Maqui-Beeren-Extrakt durch ein wässrig/alkoholisches Extraktionsmittel, insbesondere 40 : 60 (v/v) bis 60 : 40 (v/v), insbesondere 41 : 59 (v/v), oder 50 : 50 (v/v) gewonnen wird.

3. Arzneimittel oder Nahrungsergänzungsmittel zur Verwendung nach Anspruch 2, wobei das alkoholische Extraktionsmittel Ethanol, insbesondere 96 %iges Ethanol, ist.

4. Arzneimittel oder Nahrungsergänzungsmittel zur Verwendung nach einem der vorhergehenden Ansprüche, **dadurch gekennzeichnet, dass** für Schritt d.) Wasser und für Schritt e.) Ethanol verwendet wird.

5. Arzneimittel oder Nahrungsergänzungsmittel zur Verwendung nach einem der vorhergehenden Ansprüche, wobei das Pflanzenmaterial aus Schritt a.) aus Früchten ausgewählt ist.

6. Arzneimittel oder Nahrungsergänzungsmittel zur Verwendung nach einem der vorhergehenden Ansprüche, wobei das Adsorptionsmittel der Schritte c.) bis d.) ein Harz, insbesondere ein Polymerharz oder ein Polystyrolharz ist.

7. Arzneimittel oder Nahrungsergänzungsmittel zur Verwendung nach einem der vorhergehenden Ansprüche, bestehend aus einem pharmazeutischen Präparat, gegebenenfalls einschließlich geeigneter Trägersubstanzen , insbesondere in Form von Dragees, Tabletten, Filmtabletten, Pulvern, Kapseln oder flüssigen Verdünnungen, insbesondere Tropfen, Säften oder Sirupen, Salben, Emulsionen, Granulaten, Pulvern, Nasensprays, flüssigen oder festen Zubereitungen zum Inhalieren, Kompressen, Wund- und Zahnfleischverbänden, Tamponaden, Tonsillenlösungen, Gurgellösungen, Spüllösungen, Injektionen, Transfusionslösungen, Granulaten, magensaftresistenten Tabletten, Trochen, Suspensionen, Fomentationen, Nasentropfen, Inhalationsmitteln, Lotionen, Suppositorien, enteralen Nährstoffen.

## Revendications

1. Médicament ou complément alimentaire pour une utilisation dans le traitement ou la prévention d'une maladie ou d'un défaut osseux choisi dans le groupe des défauts osseux congénitaux, des déformations des os ou de la colonne vertébrale, de l'ostéosarcome, de la dysplasie osseuse, de l'ostéomalacie (rachitisme), ostéogenèse imparfaite (maladie des os fragiles), maladie de Paget, comprenant un extrait de baie de maqui, dans lequel l'extrait de baie de maqui comprend un minimum de 25 % (p/p) de delphinidines et un minimum de 35 % (p/p) d'anthocyanes, et dans lequel l'extrait est obtenu par les étapes suivantes :
a) obtention d'un extrait brut d'une matière végétale,
b) filtrer ledit extrait brut
c) mise en contact de l'extrait brut avec un adsorbant, dans lequel l'adsorbant, en particulier la résine, adsorbe les anthocyanes ;
d) lavage dudit adsorbant ; et
e) élution des anthocyanes de l'adsorbant pour obtenir une composition enrichie en anthocyanes.

2. Médicament ou complément alimentaire pour une utilisation selon l'une des revendications précédentes, dans lequel l'extrait de baie de maqui est obtenu par un agent d'extraction aqueux/alcoolique, notamment 40 : 60 (v/v) à 60 : 40 (v/v), en particulier 41 : 59 (v/v), ou 50 : 50 (v/v).

3. Médicament ou complément alimentaire pour une utilisation selon la revendication 2, dans lequel l'agent d'extraction alcoolique est l'éthanol, en particulier l'éthanol à 96 %.

4. Médicament ou complément alimentaire pour une utilisation selon l'une des revendications précédentes, dans lequel l'eau est utilisée pour l'étape d.) et l'éthanol est utilisé pour l'étape e.).

5. Médicament ou complément alimentaire pour une utilisation selon l'une des revendications précédentes, dans lequel le matériel végétal de l'étape a.) est choisi parmi les fruits.

6. Médicament ou complément alimentaire pour une utilisation selon l'une des revendications précédentes, dans lequel ledit adsorbant des étapes c.) à d.) est une résine, en particulier une résine polymère ou une résine polystyrène.

7. Médicament ou complément alimentaire pour une utilisation selon l'une des revendications précédentes comprenant une préparation pharmaceutique, le cas échéant incluant des substances porteuses appropriées , notamment sous forme de dragées, comprimés, comprimés pelliculés, poudres, gélules ou dilutions liquides, notamment gouttes, jus ou sirops, pommades, émulsions, granulés, poudres, pulvérisations nasales, préparations liquides ou solides pour inhalation, compresses, pansements pour plaies et gencives, tamponnades, solutions pour peintures d'amygdales, solutions de gargarisme, solutions de rinçage, injections, solutions pour transfusion, granules, comprimés à enrobage entérique, troches, suspensions, fomentations, gouttes nasales, inhalants, lotions, suppositoires, nutriments entéraux.
